# EUROPEAN PATENT SPECIFICATION

(11) **EP 1 808 428 B1**
(45) Date of publication and mention of the grant of the patent: **22.12.2010**
(21) Application number: 06000819.0
(22) Date of filing: 16.01.2006
(51) Int. Cl.: C07C 243/28, C07C 237/10, C23G 1/14, C23G 1/18, C23G 1/19, C02F 5/12

(54) **Descaling solutions comprising EDDH**
EDDH enthaltende Entzunderungslösungen
Solutions décapantes comprenant de l'EDDH

(43) Date of publication of application: 18.07.2007
(73) Proprietor: Research Institute of Petroleum Industry (RIPI), 18799 Tehran (IR)
(72) Inventor: Kameli, Mohammad, Farahzad Tehran (IR); Nazari, Khodadad, Sadar Jangal Blvd. Tehran (IR); Zare, Masoud, Tehran (IR)
(74) Representative: Fuchs

(56) References cited:
- EP-A- 0 290 393
- EP-A- 0 470 553
- WO-A-01/23067
- US-A- 2 480 439
- US-A- 3 308 065
- US-A- 3 351 658
- US-A- 3 580 950
- US-A1- 2004 058 853
- DATABASE CAPLUS [Online] CHEMICAL ABSTRACTS SERVICE, COLUMBUS, OHIO, US; MINAGAWA, MOTONOBU ET AL: "Stabilized halogen-containing resin compositions" XP002382571 retrieved from STN Database accession no. 1973:137421 & JP 470 393 41B B4 (SOCIETE ANON. ARGUS CHEMICAL N. V.) 7 December 1972 (1972-12-07)
- DATABASE CROSSFIRE BEILSTEIN Beilstein Institut zur Förderung der Chemischen Wissenschaften, Frankfurt am Main, DE; XP002380943 Database accession no. 2184723 (BRN) & BADINAND A. ET AL.: BULL. SOC. CHIM. FR., 1960, pages 382-383,

## Description

This invention relates to new descaling solutions and their application.

The new solutions are suited for dissolving scales.

Generally speaking, scales are dense coatings of predominantly inorganic materials formed from the precipitation of for example, water soluble constituents. Scales are often based on metals, more specifically based on transition metals or similar metals and their respective oxides, chlorides, or carbonates.

In particular, the descaling solutions according to the invention are suited to dissolve iron oxide based scales.

The descaling solutions of the invention are highly efficient for removing scales from respective industrial equipment. The new and advantageous solutions can be applied in any equipment such as industrial equipments, and their use lowers significantly corrosion when compared to the use of respective known equivalent solutions.

According to a particular embodiment of the present invention, a solution according to the invention can be advantageously used for removing scales of iron oxides and hydroxy iron oxides, in particular of a magnetite type in case of carbon steel alloys, and also insoluble salts derived from brass, solder and cast iron.

In most common industrial equipments, such as for example radiators, heat exchangers, boilers, hot water and steam pipes, heat is exchanged while water is being circulated. Under these circumstances, and also depending on the type of the circulating compound and still further of the nature of added chemicals, i.e. different additives, dissolved oxygen and carbon dioxide may lead to undesirable formation of insoluble salts and oxides on the surfaces, in particular where heat is transferred. Still further, amounts of, for example, iron, copper or other cations that may be formed as corrosion products, depending on the type of the alloy, may lead to undesired scales within the equipment.

Scales are often of a magnetite (Fe₃O₄) type, or however, also or other transition metal salts may brake off as a result of a high circulation speed or other influences, like fluid shocks, and may accumulate in different bends and behind perforated plates.

Other types of scales may also be formed in respective systems and the kind of scale depends on the regional water quality, and also factors like water hardness. Such scales may be, for example, carbonates, chlorides, oxides and sulfides of different metals, such as for example alkaline earth and transition metals. Also phosphates and silicates of metals like iron, calcium and magnesium can be formed in industrial boilers, namely as a result of the application of certain additives.

Any scale should be treated with respect to its chemical nature and physical properties. Historically speaking, most of the scale removing solutions are based on organic and inorganic acids. Among inorganic acids, acids like HCl, H₃PO₄ and sulfamic acid are used, and from an organic acid side of view, citric acid, acetic acid and ethylendiamine tetracetic acid (EDTA) are commonly used.

The application of metal (alkaline metal) and amine salts of alkene polyamine polyacetic acids for this purpose has also been reported. Phosphonic acids of HEDP and PBTC types have also been used in this respect.

All these efforts were made because the formation of such scales causes severe problems in heat exchanging with a resulting decreasing efficiency of the apparatus. Still further it is aimed at inhibiting corrosion.

U.S. patent 5,078,849 refers to formulations for dissolving iron oxide scales, based on the application of diphosphonic acids of vinyl diphosphonic and /or HEDP type and polyphosphonic acids.

Said formulations also comprise organic reducing agents, such as ascorbic acid and hydroquinone, or inorganic ones, such as sulfites, dithionites and formaldehyde sulfoxylates, which help breaking the crystalline structure of iron oxide. The acid concentration is in the range of from 1.0 to 3.0 M, that of the reducing agent being up to 5 M with the acid to reducing agent ratio being at least 10. The operating temperatures for these solutions are 80°C, and the contact time for them is in the range of from 10 minutes to 24 hours.

The major drawbacks of said solutions known from the art is an uncontrolled corrosion and still further that their application is merely limited to small parts of the system, like, for example, isolated heat exchangers of a nuclear power plant.

U.S. patent 3,996,062 discloses formulations consisting of at least 0.5% by weight of a polyphosphonic acid or its salt for dissolving iron scales.

Scales can be cleaned and removed off-line either by chemical or by mechanical means. Mechanical means show, as a whole, lots of limitations and are of low efficiency together with being both, time consuming and costly. For instance, in the case of a heavy and large locomotive radiator, it must be demounted and the plugged vessels must be cleaned.

Since with mechanical cleaning a thin layer of said precipitates will very likely remain on the surface, shortly after re-utilizing the means (such as a radiator), new scales will form and crystallize on said surfaces, even faster and stronger than before.

According to a common industrially applied method, a 10 to 25 percent solution of ((NH₄)₂H₂ EDTA (diammonium ethylene diamine tetra acetic acid), with a pH value of 7.5 or higher can be used, preferably in combination with a suited corrosion inhibitor.

Mostly, previously reported formulations use acidic solutions and in particular organic acid solutions, which may cause severe damages to the system itself. Application of metal ion complexing agents, such as hydroxyethylidene diphosphonic acids and derivatives, are alternatively suggested. This, however, is not very successful, because such solutions do not work well in removing metal oxide scales, in particular in the case of iron, and a large amount of said scales will hence remain undissolved even after washing. Most of said solutions also cause higher than standard corrosions to the alloys of the system during the cleaning process, in particular in the case of brass, carbon steel and solder.

Another drawback of known scale removing agents is that they make a total shut down of the system necessary.

Water vapor systems are gradually corroded, according to different reasons, including not using proper water or corrosion inhibitors, what may potentially lead to the formation of metal scales, in particular those of iron rust, which reduces the efficiency of the systems and can also cause plugging in the narrow channels of the systems, resulting in a partial or total shut down of the respective system.

The systems may be water circulating systems, boilers, diesel and gasoline engine radiators, etc. On the inner surfaces of such equipment metal oxide scales are potentially formed, in particular iron oxides (resulting from a corrosion processes) and also calcium and magnesium scales, which are formed as a result of water hardness.

EP 0 470 553 relates to cooling system cleaning solutions containing an EDTA salt, a polyhydroxy mono- or dicarboxylic sugar acid or sorbitol, and a soluble nitrate salt.

U.S. patent 3,351,658 refers to amino acid chelating compounds for iron. The compounds find uses in catalysis, pharmacology, chemotherapy, and industrial application in form of Schiff basis of various aldehydes; or in form of porphorine; and in agriculture. However, that document does not disclose or suggest a descaling solution.

U.S. patent 2,480,439 relates to substituted alpha amino acid amides which are of particular utility in the art as reactive extenders and modifiers for use with natural and artificial rubber, as compounds useful for further organic synthesis and in the formation of long chain high molecular weight amino compounds, and in other fields. However, that document does not disclose or suggest a descaling solution.

WO 01/23067 discloses polyamide-containing ligands covalently bonded to inorganic and organic solid supports and polyamide-containing polymeric resins. However, that document does not relate to a descaling solution.

U.S. patent application 2004/058853 refers to a multimeric compound or a pharmaceutically acceptable salt or derivative thereof which comprises three or more neuraminidase-binding groups attached to a spacer or linking group, in which the neuraminidase-binding group is a compound which binds to the active site of influenza virus neuraminidase, but is not cleaved by the neuraminidase.

EP 0 290 393 describes piperidine compounds useful as light stabilizers, heat stabilizers and oxidation stabilizers for organic materials.

Minagawa, Motononbu et al. teaches the use of amino acid amides in order to stabilize halogen-containing resin compositions (Minagawa, Motononbu et al "Stabilized halogen-containing resin compositions", Chemical Abstracts Service, Database Caplus XP 002382571).

Badinand A., et al. discloses amino acid amides (Badinand A., et al.Bull. Soc. Chim. Fr., 1960, 382-383).

U.S. patent 3,580,950 relates to chelating agents useful in washing and detergent operations. However, that document relates not to a descaling solution.

Therefore, there exists a strong need to clean and descale such systems without stopping the process, making it more economic.

However, as mentioned before, common methods of descaling make a system shut down necessary prior to circulating the solutions of inorganic acids or alkaline solutions of polycarboxylic acids such as EDTA, NTA and HEDP, which can dissolve the mentioned scales, but only when operating at relatively high temperatures. Besides the necessary system shut down, a further unwanted drawback of such methods is the occurrence of severe corrosion, and still further the whole process is energy consuming because of the necessary high temperatures.

Still further, acidic solutions, in particular those of inorganic acids, cause serious damages to the system. Complexing agents of metallic ions, such as hydroxyethylidene diphosphonic acids and their derivatives, do not act efficiently, especially in the case of iron oxides, with the consequence that major amounts of the undesired scales remain untouched. Such compounds, on the other hand, cause higher than standard corrosion in the systems, in particular in those made of or at least comprising brass, carbon steel and solder.

Summing up: When taking into consideration what is known from the art, there still exists a strong need for solutions useful for achieving optimum conditions for descaling of systems, in particular at alkaline pH values, preferably without stopping the process and/or demounting the system.

It is therefore the object of the present invention to provide new descaling solutions which are on-line useful as an efficient scale removing agent.

The scale removing solutions according to the invention are suited to significantly reduce the corrosion of systems that are descaled with said solutions, as compared to corrosion occurring with known agents.

The objects of the present invention are achieved by providing a descaling solution according to claim 1 comprising EDDH of chemical structure 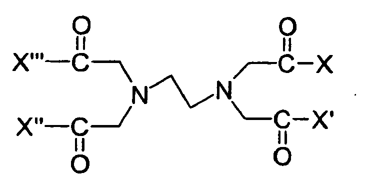 in an amount of from 0.1 to 5.0 percent by weight, which solution additionally comprises tolytriazole as a corrosion inhibitor and BOTS,
wherein *X*, *X*', *X", X"'* represent NH-NH₂.

As mentioned above, the solutions have an according to the invention improved efficiency to remove scales of metals, in particular iron scales.

Said solutions according to the invention are suited to work well at alkaline pH values and do not suffer from the drawbacks described for the acidic solutions known from the art. Said solutions are suited to dissolve iron oxide scales, what cannot be achieved with scale removing solutions that work only at neutral pH values.

According to the invention, the EDDH within the solution can be used as a chemical complexing reagent for metallic iohs. Said solutions work well at alkaline pH values of from 7 to 11, preferably of from 7.5 to 9.5 and most preferably at pH values from 7.8 to 8.4.

The EDDH according to the invention is used admixed with benzene,1,1-oxybis-tetrapropylene derivatives, being sulfonated, and in the form of a Na salt (BOTS) and tolytriazole.

The solution of the invention can be used for both, on-line and off-line descaling of systems, such as for example described above.

The EDDH used according to the invention can be produced by reacting ethylene diamine tetra acetic acid (EDTA) with hydrazine, preferably at a temperature in a range of from 80-140°C for about 10 to 30 hours.

The resulting mixture contains about 50-80%, by weight, of the target compound.

Preferably the reaction is run in such a manner that the resulting mixture does not contain any EDTA by addition of excess amounts of hydrazine as starting compounds, leading to a complete reaction of EDTA with hydrazine. Unreacted hydrazine may evaporate off the solution during the final step of the synthesis.

The product mixture comprising EDDH can preferably be used, without further separation and/or purification steps, to prepare the scale removing solutions, of the invention which still further comprise tolytriazole and BOTS.

The resulting solution is useful for removing scales comprising transitions and/or alkaline earth metal derivatives, such as transition and/or alkaline earth metal oxides, chlorides, carbonates, sulfides, phosphates, or iron oxides, in the presence of alloys like brass, carbon steel, cast iron and solder, causing no serious corrosion to the body of the system.

The concise composition of the solution may comprise different components, depending on the type of the scales and system to be cleaned..

The method for preparing a solution according to the invention is to admix the product mixture resulting from the EDDH synthesis described above in such a manner that the resulting mixture contains 0.1-5% by weight of a one or a mixure of EDDH, more preferably 2-3 % by weight, depending on the amount of scales to be removed, together with one corrosion inhibitor and a surfactant: tolytriazole, and BOTS. Preferably, the corrosion inhibitor is present in the solution in an amount of from 1 to 2,000 ppm.

The solutions work well at temperatures in the range of from 30 to 80°C, even and preferred in a range of from 35 to 50°C and most preferred in the range of from 40 to 50°C. Said solutions descale the apparatus in an optimum time frame of 2-10 hours.

Said solutions may still further comprise biodegradable oxygen or nitrogen containing organic compounds. After the cleaning process the used solution may comprise metal complexes that can be usefully absorbed by plants as micronutrients, as long as the cleaned equipment does not contain lead (Pb) or cadmium (Cd).

The descaling solutions in accordance with the invention are alkaline and hence do not suffer from the disadvantages of acidic solutions which cause corrosion. These solutions can in particular dissolve iron oxides, while other scale removing solutions, which work at neutral pH values (-6.5) lack this ability. Solutions comprising one or more of the compounds used according to the invention do not damage the systems because they do not cause severe corrosions in the system. For such new descaling solutions even less amounts of the active compound (2 to 5% wt) as compared to the solutions discussed in the art (making the use of up to 30% wt of an active compound) are necessary. Hence, the solutions comprising one or more compound, according to the invention are much more economical.

The following data and examples are presented to describe the invention in more detail:

The further examples presented below refer to descaling experiments with different descaling solutions known from the art as well as to respective experiments with descaling solutions comprising EDDH according to the invention prepared in a manner suited for the respective scale to be removed. The results are then compared.

The following experiments are performed with a locomotive radiator, and the respective descaling formulations are adjusted for this purpose.

### Example 1: Descaling by polycarboxylic acids (comparative example)

A 34 % wt EDTA solution, having a pH of 11 was prepared, and rusted (oxidized) carbon steel coupons (already prepared through the so-called salt spray method) were placed in 500 ml of the solution. After stirring the mixture for several days, the scales on the coupons were still present, i.e. have not been descaled.

One can conclude that an EDTA solution, having a pH of 11, cannot be used to remove iron oxide scales. Even lowering the pH to 5.0 or a little lower has not been suited to remove iron oxide based scales. This is partially due to limited solubility of EDTA at these pH ranges.

Similar tests were performed using EDTA solutions with concentrations of from 10 to 50% wt, at, both room temperature and 80°C. Also the results from such experiments confirm that EDTA solutions do not have considerable descaling properties, in particular for iron oxides based scales.

### Example 2: Descaling by HEDP solutions (comparative example)

To observe the cleaning power of hydroxy ethylidene diphosphonic acid (HEDP), a 34% wt solution with a pH of 5.0 was prepared and a rusted coupon was put in it for 72 hours, at room temperature. One could observe that some scales were removed. However, the solution contained some undissolved scale flakes, and the coupons had turned black.

A 34% wt solution of HEDP having a less acidic pH of 6.5 was difficult to achieve due to the precipitation of tri sodium HEDP salt.

0.25, 0.5, 2,0 and 5.0% wt solutions of HEDP were prepared by diluting a 10% wt stock solution and the rusted coupons were placed in the said diluted solutions. In all cases the scales came off to a great extent but the solution containd a large amount of undissolved precipitates and the coupons were intensively corroded.

To evaluate the descaling power of HEDP solutions on radiators, the rate of the HEDP-induced corrosion of carbon steel coupons were studied and the results are given in following table:

| **Descaling solution based on 2% HEDP** | **Corrosion rate im mpy*** |
|---|---|
| Without a corrosion inhibitor | 63.6 |
| In presence of a corrosion inhibitor | 61.4 |

| | |
|---|---|
| *mil per year (1 mil = 1/1000 inch) | |

Regarding the HEDP-induced corrosion, it is high but still the solution does not have a sufficient descaling effect. That leads to the conclusion that the compound is not suited for descaling of, for example, radiators.

### Example 3: Effect of pH and temperature on scale removal by EDDH solutions

To investigate the optimum conditions for descaling, different solutions comprising of from 0.1% to 5.0% wt of EDDH with different pH values were prepared.

As a result it was observed that at room temperature, 5% wt solutions work relatively better than the other solutions at low pH values. However, one has to consider that 5% wt solutions as compared to 2%wt solutions lead to a higher corrosion rate. As a matter of principle, the speed of scale removal becomes faster as the concentration and temperature are increased.

### Example 4: Controlled descaling in the presence of BOTS and Tolytriazole according to the present invention

To optimize the scale-removing rate while maintaining an acceptable corrosion rate, various additives were examined.
a) A 2% wt solution of EDDH at pH of 8 and 50°C was used for carbon steel, brass, solder and cast iron coupons and the following results were obtained:

| **Coupon alloy** | **Corrosion rate (mpy)** |
|---|---|
| Carbon steel | 238 |
| Brass | 32 |
| Solder | 596 |
| Cast iron | 117 |

The time needed for proper descaling the coupons in the mentioned test was found to be 1.5 hours.
b) A 2% wt solution of EDDH comprising 100-500 ppm of the corrosion inhibitor tolytriazole and 800-1500 ppm of BOTS, were prepared and used at 50°C. The following results were obtained.

| **Coupon alloy** | **Corrosion rate at pH=8.0 (mpy)** | **Corrosion rate at pH=9 (mpy)** |
|---|---|---|
| Carbon steel | 9.0 | 14.5 |
| Brass | 3.0 | 3.2 |
| Solder | 34.8 | 8.8 |
| Cast iron | 25.0 | 30.4 |

The time needed for cleaning the coupons was 10 hours at pH=8.0 and 18 hours at pH=9.0.
c) A 2% wt solution of EDDH comprising 10-150 ppm of the corrosion inhibitor tolytriazole, and 800-1500 ppm of BOTS was prepared and used for the same coupons at 50°C, with the following results:

| **Coupon alloy** | **Corrosion rate (mpy)** |
|---|---|
| Carbon steel | 6.6 |
| Brass | 1.3 |
| Solder | 27.0 |
| Cast iron | 9.0 |

The time required for cleaning the coupons was 10 hours.
d) A 2% wt solution of EDDH comprising 10-150 ppm of the corrosion inhibitor, a tolytriazole, and 800-1500 ppm of BOTS together with at least 20 ppm of terpolymer was prepared, and said solution, having a pH of 8, was used to descale the same coupons at 50°C.

| **Coupon alloy** | **Corrosion rate (mpy)** |
|---|---|
| Carbon steel | 15.8 |
| Brass | 5.5 |
| Solder | 23.7 |
| Cast iron | 30.0 |

Since terpolymer disperses the particulate matters such that the chelant can effectively attack the fresh surfaces of scale, this solution even works better than the one described under c).

The time needed for cleaning the coupons in this case was 10 hours.

From the results shown above, optimum conditions could be achieved with the solution according to Example 4d.

Starting with that solution, for example the amount of BOTS was modified to 80-125 ppm for static descaling, showing good effects.

### Example5: Dynamic descaling, using a dynamic corrosion inhibitor evaluation device

This example aims at finding optimum conditions for solutions according to the invention in industrial systems.

A dynamic corrosion and descaling evaluation device, which is suited to simulate the required conditions in industrial systems was used.

Adjusting variables like temperature, pH, water circulation rate and also concentration of corrosion inhibitors and further additives, tests were performed under the following conditions:
a) A 2% wt descaling solution (i.e. comprising 2 % by weight of EDDH), still further comprising 800-1500 ppm of BOTS and 80-200 ppm of the corrosion inhibitor tolytriazole, and 400-600 ppm of the reducing agent Na₂SO₃, having a pH of 8 was prepared and used at 40°C, for 3 hours:

| **Coupon alloy** | **Corrosion rate (mpy)** |
|---|---|
| carbon steel | 118.0 |
| brass | 8.6 |
| Solder | 143.0 |
| cast iron | 209.0 |

One can conclude from the results, that the cleaning efficacy is perfect under such conditions.
b) A 2% wt descaling solution (i.e. comprising 2 % by weight of EDDH), optionally also comprising 20-40 ppm of BOTS, 800-1500 ppm of the corrosion inhibitor tolytriazole, and 10-50 ppm of another corrosion inhibitor, most preferably of tri deca-benzemidazole type, and 10-50 ppm of a third corrosion inhibitor which is preferred to be a lipophile amine of THTA type was prepared and used at 40°C in the following manner:
1- At first, the 2% wt descaling solution was used without any additives for 20 minutes at a pH of from 6 to 7, during which the rusted coupons were cleaned up to 80%.
2- After this step, the mentioned additives and corrosion inhibitors were added to said initial solution, and the descaling was continued with the solution, now having a pH of from 7.5 to 8.2, until a total test time of 5.5 hours passed.
The results are:

| **Coupon alloy** | **Corrosion rate (mpy)** |
|---|---|
| Carbon steel | 196.0 |
| Brass | 10.8 |
| Solder | 73.0 |
| Cast iron | 550 |

Under such condition, the descaling efficiency again was very high.
c) A 2% wt. descaling solution of EDDH comprising furthermore 80-120 ppm of the corrosion inhibitor tolytriazole, 10-40 ppm of BOTS, 1-10 ppm of another corrosion inhibitor of a THTA type, and 5-18 ppm of Terpolymer, having a pH value of from 7.5 to 8.3 was prepared and used for 8.4 hours at 40°C.

The results are:

| **Coupon alloy** | **Corrosion rate (mpy)** |
|---|---|
| Carbon steel | 3.6 |
| Brass | 1.4 |
| Solder | 2.6 |
| Cast iron | 165.0 |

Under these conditions the descaling ability is high.

### Example 6: Descaling of a locomotive radiator

To be descaled was a locomotive radiator with high amounts of scales present. 200 liters of a 2% descaling solution (comprising EDDH), which solution still further comprises 80-130 ppm of the corrosion inhibitor tolytriazole, 10-45 ppm of BOTS 1-10 ppm of another corrosion inhibitor, THTA (Tris(2-hydroxyethyl)N-tallow alkyl diaminopropane and 1-15 ppm of terpolymer (a carboxylate/sulfonate/nonionic terpolymer) having a pH of 8, was used for 4 hours, at 40°C:

The results are:

| **Coupon alloy** | **Corrosion rate (mpy)** |
|---|---|
| Carbon steel | 11.6 |
| Brass | 0.86 |
| Solder | 16.0 |
| Cast iron | 43.0 |

Under these conditions, the descaling efficiency of the solution was very high, and no scale was left on the respective parts of the radiator. Said descaling solution can dissolve iron efficiently, even with a 1:1 stoichiometric ratio, which is equivalent to the dissolution of 7.2 grams of dry scale by one litre of the 2% descaling solution.

According to the laboratory tests, a time period of 4 hours and a temperature in the range of from 40 to 45°C showed to be optimum conditions for the descaling process.

A properly designed, side stream filter or main line filter can easily remove most of the flaked-off scales, thus saving in chemical costs of on-line cleaning. Static tests with EDDH solutions according to the invention showed that for systems (specially those not containing solder), the water circulation rates of which are not very high, optimum conditions are:

2% wt of EDDH, more preferably a 5% wt in solution comprising 80 to 150 ppm of the corrosion inhibitor of the tolytriazole type, 85 to 125 ppm of the surfactant, BOTS are used at 50°C and for 10 hours.

On the other hand, the results obtained from the dynamic tests of the scale removing solution reveal that in systems with high water circulation rates the following conditions can be considered as suited:

2% wt of EDDH in solution, comprising 80 to 130 ppm of the corrosion inhibitor of the tolytriazole type, 20 to 40 ppm of the surfactant BOTS, 1 to 10 ppm of a second corrosion inhibitor, most preferably of the THTA type and 1 to 15 ppm of Terpolymer are used at 42-45°C with a pH of 8 for 4 hours. These conditions are in particular proper for systems not including cast iron.

Given that the liquid flow rates are high in radiators, applying these conditions in a radiator-descaling test proved them to be appropriate for such applications. It seems that high flow rates tend to increase corrosion. Hence, considerable decrease of the flow rate in the radiator compared to that of the evaluation apparatus reduces cast iron corrosion from 165 mpy to 43 mpy.

For descaling of radiators a temperature optimum range is from 40 to 50°C.

It is also most preferred to take out the loose scales plugging different parts of the radiator to perform an on-line descaling.

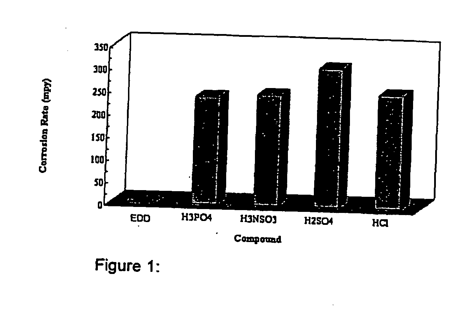

## Claims

1. A descaling solution comprising EDDH, **characterized in that** EDDH is present in an amount of from 0.1 to 5.0 percent by weight and the solution additionally comprises tolytriazole as a corrosion inhibitor and sulfonated benzene-1,1-oxybis-tetrapropylene or its derivatives as sodium salts (BOTS).

2. The descaling solution according to claim 1, wherein tolytriazole is present in amounts of from 1 to 2000 ppm.

3. The descaling solution according to one or more of the preceding claims, wherein the pH value of the solution is between 7 and 10.

4. The descaling solution according to one or more of the preceding claims, wherein the solution has a pH of from 7.5 to 9.5.

5. The descaling solution according to one or more of the preceding claims, wherein the solution has a pH of from 7.8 to 8.4.

6. A process for removing scales from industrial equipment, comprising the step of
a. applying to the equipment a descaling solution according to one or more of claims 1 to 5.

7. A process for removing scales from industrial equipment, comprising the step of
a. applying to the equipment a descaling solution according to one or more of claims 1 to 5 at a temperature of between 30 to 80°C.

8. Process for removing scales from industrial equipment according to claims 6 or 7, comprising the step of
a. applying to the equipment a descaling solution according to one or more of claims 1 to 5 for a period of between 2 to 10 hours.

## Patentansprüche

1. Eine entkalkende Lösung, die EDDH umfasst und **dadurch gekennzeichnet ist, dass** EDDH in einer Menge von 0,1 bis 5,0 Gew.-% vorhanden ist und die Lösung zusätzlich Tolytriazol als Korrosionsinhibitor und sulfoniertes Benzol-1,1-oxybis-tetrapropylen oder dessen Derivate als Natriumsalz (BOTS) umfasst.

2. Die entkalkende Lösung gemäß Anspruch 1, wobei Tolytriazol in einer Menge von 1 bis 2000 ppm vorhanden ist.

3. Die entkalkende Lösung gemäß einem oder mehreren der vorhergehenden Ansprüche, wobei der pH Wert der Lösung zwischen 7 und 10 ist.

4. Die entkalkende Lösung gemäß einem oder mehreren der vorhergehenden Ansprüche, wobei die Lösung einen pH Wert von 7,5 bis 9,5 hat.

5. Die entkalkende Lösung gemäß einem oder mehreren der vorhergehenden Ansprüche, wobei die Lösung einen pH Wert von 7,8 bis 8,4 hat.

6. Ein Verfahren zum Entfernen von Kalk von industriellen Gerätschaften, umfassend den Schritt:
a. Anwenden einer entkalkenden Lösung gemäß einem oder mehreren der Ansprüche 1 bis 5 an den Gerätschaften.

7. Ein Verfahren zum Entfernen von Kalk von industriellen Gerätschaften, umfassend den Schritt:
a. Anwenden einer entkalkenden Lösung gemäß einem oder mehreren der Ansprüche 1 bis 5 an den Gerätschaften bei einer Temperatur im Bereich zwischen 30 bis 80 °C.

8. Verfahren zum Entfernen von Kalk von industriellen Gerätschaften gemäß den Ansprüchen 6 oder 7, umfassend den Schritt:
a. Anwenden einer entkalkenden Lösung gemäß einem oder mehreren der Ansprüche 1 bis 5 an Gerätschaften für eine Dauer von 2 bis 10 Stunden.

## Revendications

1. Solution décapante comprenant de l'EDDH, **caractérisée en ce que** l'EDDH est présent en une quantité de 0,1 à 5,0 pour cent en poids et la solution comprend de plus du tolytriazole comme inhibiteur de corrosion et du benzène-1,1-oxybis-tétrapropylène sulfonaté ou ses dérivés comme sels de sodium (BOTS).

2. Solution décapante selon la revendication 1, dans laquelle le tolytriazole est présent en des quantités allant de 1 à 2 000 ppm.

3. Solution décapante selon une ou plusieurs des revendications précédentes, dans laquelle la valeur de pH de la solution est comprise entre 7 et 10.

4. Solution décapante selon une ou plusieurs des revendications précédentes, dans laquelle la solution a un pH de 7,5 à 9,5.

5. Solution décapante selon une ou plusieurs des revendications précédentes, dans laquelle la solution a un pH de 7,8 à 8,4.

6. Procédé d'élimination de calamine d'un équipement industriel, comprenant l'étape consistant à :
a. appliquer à l'équipement une solution décapante selon une ou plusieurs des revendications 1 à 5.

7. Procédé d'élimination de calamine d'un équipement industriel, comprenant l'étape consistant à :
a. appliquer à l'équipement une solution décapante selon une ou plusieurs des revendications 1 à 5 à une température comprise entre 30 et 80°C.

8. Procédé d'élimination de calamine d'un équipement industriel, selon les revendications 6 ou 7, comprenant l'étape consistant à :
a. appliquer à l'équipement une solution décapante selon une ou plusieurs des revendications 1 à 5 pendant une période comprise entre 2 et 10 heures.
